(19) Europäisches Patentamt
European Patent Office
Office européen des brevets

(11) Veröffentlichungsnummer: **0 230 985**
**B1**

# (12) EUROPÄISCHE PATENTSCHRIFT

(45) Veröffentlichungstag der Patentschrift: **01.08.90**

(51) Int. Cl.⁵: **C07C 245/06,** C07D 333/36, C07D 277/50, C07D 275/04, C07K 5/06, G01N 33/573

(21) Anmeldenummer: **87100836.3**

(22) Anmeldetag: **22.01.87**

## (54) Neue Gamma-Glutamyl-4-azoanilide, Verfahren zur Herstellung dieser Verbindungen sowie deren Verwendung zur Bestimmung von Gamma-Glutamyltransferase.

(30) Priorität: **28.01.86 DE 3602443**

(43) Veröffentlichungstag der Anmeldung: **05.08.87 Patentblatt 87/32**

(45) Bekanntmachung des Hinweises auf die Patenterteilung: **01.08.90 Patentblatt 90/31**

(84) Benannte Vertragsstaaten: **AT BE CH DE ES FR GB GR IT LI LU NL SE**

(56) Entgegenhaltungen:
**PATENT ABSTRACTS OF JAPAN, Band 5, Nr. 168 (C-77)[840], 27. Oktober 1981; & JP - A - 56 97260 (TOYO BOSEKI K.K.) 05-08-1981**
**PATENT ABSTRACTS OF JAPAN, Band 6, Nr. 31 (C-92)[909], 24. Februar 1982; & JP - A - 56 148 299 (TOYO BOSEKI K.K.) 17-11-1981**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Sandhofer Strasse 116, D-6800 Mannheim 31(DE)**

(72) Erfinder: **Rauscher, Elli, Dr.rer.nat., Guardinistrasse 71, D-8000 München 70(DE)**
Erfinder: **Griffiths, John Dr., 9 Ashlea Close, Garforth Leeds LS25 lJX,(GB)**
Erfinder: **Dixon, Leonard Fox, 11 Bowood Road, Elland W.Yorkshire, HX5 0NW(GB)**

EP 0 230 985 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der Erfindung sind neue γ-Glutamyl-4-azoanilide, ein Verfahren zur Herstellung dieser Verbindungen sowie deren Verwendung als Substrat zur Aktivitätsbestimmung der γ-Glutamyltransferase.

Die Bestimmung der γ-Glutamyltransferase (γ-GT) wird im klinisch-chemischen Labor zur Diagnostik von Lebererkrankungen durchgeführt. Nach einem bekannten Verfahren erfolgt die Bestimmung entsprechend der Reaktion:

γ-Glutamyl-p-nitroanilid +Glycylglycin ⇄ (γ-GT) γ-Glutamylglycylglycin + p-Nitroanilin

Die Geschwindigkeit der Freisetzung des gelbgefärbten p-Nitroanilins kann optisch verfolgt werden. Sie ist ein Maß für die vorhandene Aktivität an γ-GT.

Für eine routinemäßige Anwendung dieses Verfahrens sind die Instabilität und die schlechte Löslichkeit des Substrates γ-Glutamyl-p-nitroanilid nachteilig.

Diese Nachteile können durch Verwendung der in DE-PS 22 59 512 oder DE-PS 23 33 798 beanspruchten γ-Glutamyl-4-nitroanilid-Derivate, die in 3-Position einen Carbonsäure- bzw. Sulfonsäurerest tragen, weitgehend vermieden werden. Die Absorptionsmaxima dieser Verbindungen sowie der durch enzymatische Spaltung mit γ-GT entstehenden Anilinderivate liegen jedoch weit unter 400 nm.

In diesem Wellenlängenbereich stört bereits merklich die Eigenabsorption von Inhaltsstoffen der Proben, beispielsweise Bilirubin oder trübungsverursachende Stoffe.

Aus Basic Abstracts Journal (Derwent Publications Ltd.), Section B, Week D 38 vom 11.11.1981, Abstract Nr. 68 890 D/38 und Section B, Week D 52 vom 24.02.1982, Abstract Nr. 96 014 D/52, in denen die japanischen Patentanmeldungen 56-097 260 und 56-148 299 referiert sind, sind γ-Glutamyl-p-azoanilide der Formel

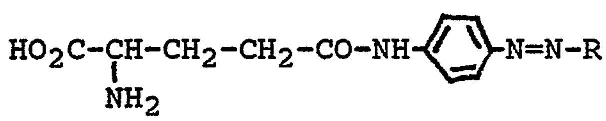

bekannt, wobei R einen Phenyl- oder Naphthylrest darstellt, der durch Wasserstoff, Methyl, Hydroxy, Dimethylamino, Diethylamino oder Nitro und durch eine Säuregruppe oder deren Salz substituiert ist. Die Differenz der Absorptionsmaxima für beispielsweise das entsprechende p-Nitrophenyl-substituierte γ-Glutamyl-p-azoanilid und dem durch Abspaltung der γ-Glutamylgruppe entstehenden Azofarbstoff beträgt etwa 50 nm. Wünschenswert sind γ-GT-Substrate mit möglichst großen Absorptionsmaxima-Differenzen zwischen γ-GT-Substrat und durch Enzymeinwirkung resultierendem Farbstoff, da dadurch empfindlichere Tests ermöglicht werden.

Es bestand daher Bedarf an γ-GT-Substraten, welche die genannten Nachteile nicht aufweisen. Aufgabe der vorliegenden Erfindung war es, diesen Bedarf zu befriedigen.

Gelöst wird diese Aufgabe durch die erfindungsgemäßen neuen γ-Glutamyl-4-azoanilide der allgemeinen Formel I:

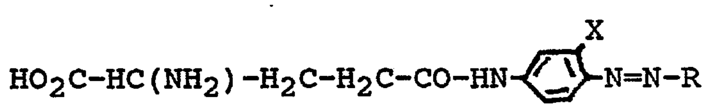
(I)

in der

X eine Gruppe $-(CH_2)_m-CO_2H$, $-O(CH_2)_n-CO_2H$ oder einen Alkylrest,

R eine p-Nitrophenylgruppe, die gegebenenfalls ein- oder mehrfach durch Halogen, einen Cyanorest, einen Alkoxyrest, eine Hydroxygruppe, einen Alkylrest, einen Carboxylalkylrest, eine Aminogruppe, einen Alkylamino- oder Dialkylaminorest substituiert sein kann, wobei die Substituenten gleich oder verschieden sein können,

ein Thiophenrest, der gegebenenfalls ein- oder mehrfach durch einen Cyano-, Nitro-, Alkyl- oder Carboxylrest substituiert sein kann, wobei die Substituenten gleich oder verschieden sein können,

ein Thiazolrest, der gegebenenfalls ein- oder mehrfach durch einen Cyano-, Carboxyl- oder Alkylrest substituiert sein kann, wobei die Substituenten gleich oder verschieden sein können,

einen Benzothiazolrest, der gegebenenfalls ein- oder mehrfach durch einen Alkoxyrest substituiert sein kann,

einen Benzoisothiazolrest, der gegebenenfalls ein- oder mehrfach durch eine Nitrogruppe substituiert sein kann oder

einen N-Alkylthiazolrest oder
einen 1,3,4-Thiodiazolrest, der gegebenenfalls ein- oder mehrfach durch eine Alkylthiogruppe substituiert sein kann
bedeuten, wobei
m eine ganze Zahl von 0 bis 4 und
n eine ganze Zahl von 1 bis 4 darstellen,
sowie deren Alkali-, Erdalkali- und Ammoniumsalze.

Alkalisalze können hierbei Lithium-, Natrium-, Kalium-, Rubidium- oder Cäsiumsalze sein. Bevorzugt sind Lithium-, Natrium- und Kaliumsalze.

Unter Erdalkalisalzen sind Magnesium-, Calcium-, Strontium- oder Bariumsalze zu verstehen. Bevorzugt sind Magnesium- und Calciumsalze.

Ammoniumsalze können unsubstituierte Ammoniumsalze sein oder auch solche, die ein-, zwei-, dreioder vierfach durch Alkyl- oder Aralkylreste substituierte Ammoniumionen enthalten, wobei die Substituenten gleich oder verschieden sein können. Alkylreste können hierbei Reste mit 1 - 7, bevorzugt 1 - 4 Kohlenstoffatomen sein. Bevorzugter Aralkylrest ist die Benzylgruppe. Besonders bevorzugtes Ammoniumion ist das unsubstituierte Ammoniumion.

Halogen als Substituent eines Restes R bedeutet Fluor, Chlor, Brom oder Jod. Bevorzugt sind jedoch vor allem Chlor und Brom.

Unter Alkyl- bzw. Alkoxyresten in der Definition der Substituenten der Reste R und X werden Reste aus 1 bis 7, bevorzugt 1 bis 4 Kohlenstoffatomen verstanden. Besonders bevorzugt sind der Methyl- oder Ethyl- bzw. Methoxy- oder Ethoxyrest.

Die für Alkylgruppen genannte Bedeutung gilt auch für die in Carboxyalkyl-, Alkylthio-, Alkylamino- oder Dialkylaminoresten vorkommenden Alkylgruppen, wobei bei Dialkylaminoresten die Alkylsubstituenten sowohl gleich als auch verschieden sein können. Ebenso hat auch der im N-Alkylthiazolrest vorhandene Alkylsubstituent die oben angeführte Bedeutung.

Die erfindungsgemäßen Verbindungen weisen überraschenderweise neben guter Wasserlöslichkeit und Stabilität in wässriger Lösung eine große Differenz der Absorptionsmaxima von Substrat und dem durch die Einwirkung von γ-GT freigesetzten Farbstoff auf. So beträgt beispielsweise die Differenz der Absorptionsmaxima von Substrat- und Farbstoffspektrum bei
γ-Glutamyl-3-carboxy-4-nitroanilid ($\lambda_{max}$ 317 nm) und
3-Carboxy-4-nitroanilin ($\lambda_{max}$ 380 nm) 63 nm.

Die erfindungsgemäßen Verbindungen der allgemeinen Formel I weisen dagegen wesentlich größere Unterschiede zwischen den Maxima der Absorptionsspektren von Substrat und enzymatisch freigesetztem Farbstoff auf. Sie betragen in der Regel etwa 80 - 110 nm.

Außerdem absorbieren die bei der Einwirkung von γ-GT auf die erfindungsgemäßen γ-Glutamyl-4-azoanilide freigesetzten Farbstoffe in einem wesentlich längerwelligen Bereich als bisher bekannte vergleichbare Verbindungen. γ-GT-Bestimmungen mit diesen Verbindungen unterliegen deshalb weniger Störungen in biologischen Proben.

Besonders bevorzugte Verbindungen sind:

2-Amino-5-[[4-)2-cyan-4-nitrophenyl)-azo-3-(2-carboxyethyl)phenyl]-amino]-5-oxopentansäure, Diammoniumsalz
$\lambda_{max}$ = 394 nm
$\Delta\lambda_{max}$ = 94 nm

2-Amino-5-[[3-(2-carboxyethyl)-4-(2,4-dicyan-3-methyl-5-thienyl)-azophenyl]amino]-5-oxopentansäure, Diammoniumsalz
$\lambda_{max}$ = 424 nm
$\Delta\lambda_{max}$ = 106 nm

2-Amino-5-[[3-(2-carboxyethyl)-4-(5-cyan-4-methyl-2-thiazolyl)-azophenyl]-amino]-5-oxopentansäure, Diammoniumsalz
$\lambda_{max}$ = 420 nm
$\Delta\lambda_{max}$ = 100 nm

2-Amino-5-[[3-carboxymethoxy-4-(6-nitro-2,1-benzisothiazol-3-yl)-azophenyl]amino]-5-oxopentansäure, Diammoniumsalz
$\lambda_{max}$ = 453 nm
$\Delta\lambda_{max}$ = 107 nm

$\Delta\lambda_{max}$ bedeutet hierbei die Differenz
$\lambda_{max}$(Farbstoff) -$\lambda_{max}$(Substrat).

Die erfindungsgemäßen γ-Glutamylderivate der allgemeinen Formel I sind neue Verbindungen.

Die Herstellung dieser Verbindungen ist ebenfalls Gegenstand der Erfindung. Sie erfolgt durch Umsetzung von bekannten oder nach üblichen Methoden leicht herstellbaren Aminen der allgemeinen Formel II

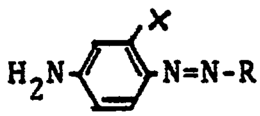

(II)

in der X und R die in der allgemeinen Formel I angegebene Bedeutung haben, mit N-Phthaloylglutaminsäureanhydrid

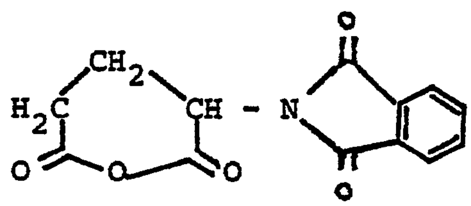

in an sich bekannter Weise zu N-Phthaloylgeschützten γ-Glutamyl-Derivaten der Azofarbstoffe der allgemeinen Formel III

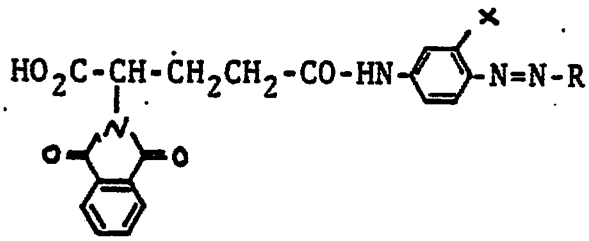

(III)

in der X und R die in den allgemeinen Formeln I und II angegebene Bedeutung haben und anschließende Entfernung der N-Phthaloyl-Schutzgruppe. Die hierbei entstehenden Carbonsäuren können anschließend nach bekannten Methoden in gewünschte Salze überführt werden. Je nach gewählten Verfahrensbedingungen können auch direkt die Salze erhalten werden.

Die Umsetzung von Aminen der allgemeinen Formel II mit N-Phthaloyl-glutaminsäureanhydrid kann in niederen aliphatischen Carbonsäuren, wie z. B. Ameisen- oder Essigsäure, erfolgen. Bevorzugt wird Essigsäure verwendet. Zur Umsetzung relativ reaktiver Amine der allgemeinen Formel II kann die als Lösungs- und Aktivierungsmittel eingesetzte aliphatische Carbonsäure auch mit anderen polaren, aprotischen Lösungsmitteln, wie z. B. Aceton, Pyridin, Dimethylsulfoxid oder Dimethylformamid, verdünnt sein. Besonders bevorzugt ist Aceton. Im Falle besonders reaktiver Amine der allgemeinen Formel II genügt es auch, die Umsetzung mit N-Phthaloylglutaminsäureanhydrid in polaren, aprotischen Lösungsmitteln durchzuführen, die nicht mit Aminen und Anhydriden reagieren. Als besonders geeignet hat sich hier ebenfalls Aceton erwiesen. Lösen sich Amine in Aceton als alleinigem Lösungsmittel nur schlecht, kann die Löslichkeit besonders vorteilhaft durch Zugabe von Pyridin erhöht werden.

Das zur gewünschten Umsetzung erforderliche Mengenverhältnis von Amin zu Anhydrid hängt von der Reaktivität der eingesetzten Amine der allgemeinen Formel II ab. Es kann von äquimolar bis zu 100fachem Überschuß an Anhydrid reichen. Vorteilhafterweise wird ein molares Verhältnis von 1:1 bis 10:1 von Anhydrid : Amin eingesetzt. Das erforderliche Verhältnis kann jeweils leicht von jedem Fachmann ermittelt werden: Die Reaktionstemperatur kann etwa 20 bis etwa 120° C betragen. Sie hängt von der Reaktivität des Amins und dem verwendeten Lösungsmittel ab. So erfolgen Umsetzungen in Aceton als Lösungsmittel vorteilhafterweise bei 20 bis 60° C, besonders bevorzugt bei 25 - 40° C. Reaktionen in Essigsäure als Lösungsmittel werden bei Temperaturen von 50 - 120° C, bevorzugt unter Rückfluß des Lösungsmittels durchgeführt. Optimales Mengenverhältnis der Reaktionspartner, Reaktionstemperatur und Reaktionszeit können auf einfache Weise durch dünnschichtchromatographische Verfolgung der Reaktion auf Kieselgel ermittelt werden.

Nach beendeter Reaktion kristallisiert in vielen Fällen das Produkt der allgemeinen Formel III aus. Erfolgt keine Kristallisation, kann das Produkt chromatographisch an Kieselgel gereinigt werden.

Zur Herstellung der erfindungsgemäßen γ-Glutamylderivative der allgemeinen Formel I muß die N-Phthaloyl-Schutzgruppe der Verbindungen der allgemeinen Formel III entfernt werden. Diese Umsetzung kann an isolierten und gereinigten Verbindungen der allgemeinen Formel III durchgeführt werden, aber auch mit dem Rohprodukt, das aus der Reaktionsmischung zur Herstellung dieser Verbindungen durch Entfernung des Lösungsmittels erhalten wird.

Die Entfernung der N-Phthaloyl-Schutzgruppe kann nach verschiedenen Methoden erfolgen. Als besonders vorteilhaft hat sich die Verwendung von Hydrazin erwiesen. Hierzu wird N-Phthaloylglutamyl-Azofarbstoff der allgemeinen Formel III in so viel niederem Alkohol, bevorzugt Methanol, suspendiert oder gelöst, daß nach Beendigung der Reaktion der gesamte Azofarbstoff in Lösung vorliegt. Anschließend wird bei Raumtemperatur Hydrazinhydrat zugegeben. Zeigt dünnschichtchromatographische Kontrolle auf Kieselgel, daß die Reaktion beendet ist, wird die Mischung mit einem Keton versetzt, um überschüssiges Hydrazin zu binden. Bevorzugt wird hierzu Aceton verwendet. Zu der klaren Lösung wird dann soviel wässrige Ammoniaklösung gegeben wie erforderlich ist, um die N-Glutamylderivate der allgemeinen Formel I als Ammoniumsalze auszufällen. Erfolgt die Ausfällung nur unvollständig, kann Aceton so lange zugegeben werden, bis die Fällung vollständig ist.

Die Reinigung der so erhaltenen Substanzen kann entweder durch Umkristallisation in geeigneten Lösungsmitteln, wie z. B. Wasser- Aceton erfolgen oder durch Chromatographie an Kieselgel.

Ein weiterer Gegenstand der Erfindung ist die Verwendung der erfindungsgemäßen Verbindungen der allgemeinen Formel I als Substrate zur Bestimmung der Aktivität des Enzyms γ-Glutamyltransferase (γ-GT).

Ferner werden diagnostische Mittel zur Bestimmung der Aktivität von γ-GT beansprucht, welche die erfindungsgemäßen Verbindungen der allgemeinen Formel I enthalten.

Die Bestimmung der Aktivität von γ-GT kann mit den erfindungsgemäßen Verbindungen der allgemeinen Formel I, beispielsweise entsprechend folgender Reaktion durchgeführt werden:

$$\text{(I)} + \text{Peptid} \underset{}{\overset{\gamma\text{-GT}}{\rightleftharpoons}} \text{(II)} + \gamma\text{-Glutamylpeptid}$$

γ-GT und Verbindungen der allgemeinen Formel I reagieren in wässriger Lösung unter Freisetzung eines Azofarbstoffes der allgemeinen Formel II und Übertragung des γ-Glutamylrestes auf eine Aminosäure oder ein Peptid. Die durch die Bildung des Farbstoffes (II) sich ergebende Extinktionszunahme kann bei einer entsprechenden Wellenlänge bestimmt werden. Ihre Geschwindigkeit ist ein Maß für die Aktivität des Enzyms.

Die Verwendung erfindungsgemäßer Verbindungen der allgemeinen Formel I als Substrate für γ-GT führt aufgrund der größeren Differenzen der Absorptionsmaxima von Substrat und enzymatisch freigesetztem Farbstoff und außerdem der Absorption des entstehenden Azofarbstoffes bei wesentlich höheren Wellenlängen zu empfindlicheren Testsystemen, als sie bisher bekannt sind. Die neuen Substrate können zur Bestimmung der Aktivität von γ-GT sowohl im biochemischen als auch im klinisch-chemischen Bereich eingesetzt werden.

Diagnostische Mittel, die γ-Glutamyl-4-azoanilide der allgemeinen Formel I enthalten, zeigen demnach erhebliche Vorteile gegenüber dem bekannten Stand der Technik. Die höhere Empfindlichkeit führt zu einer Senkung der Nachweisgrenze für γ-GT, zu kürzeren Reaktionszeiten und zu geringerem Probeneinsatz und damit auch zu geringeren Störungen durch andere Probenbestandteile. Günstigere Meßwellenlängen vermindern außerdem die Störanfälligkeit der Methode durch unlösliche Bestandteile, beispielsweise durch Trübungen.

Das diagnostische Mittel enthält neben einen oder mehreren der erfindungsgemäßen Substrate der allgemeinen Formel I, eine Aminosäure oder ein Peptid als Akzeptor des γ-Glutamylrestes, ein geeignetes Puffersystem sowie gegebenenfalls weitere geeignete, üblicherweise für solche diagnostische Mittel verwendete Zusatzstoffe, wie beispielsweise Netzmittel, Stabilisatoren etc. Das diagnostische Mittel kann in Form einer Lösung, als Lyophilisat, als Pulvergemisch, Reagenztablette oder auf einen saugfähigen Träger aufgezogen vorliegen.

Das erfindungsgemäße diagnostische Mittel in Form einer Lösung enthält vorzugsweise sämtliche für den Test benötigten Reagentien. Als Lösungsmittel kommen Wasser oder Gemische von Wasser mit einem wasserlöslichen organischen Lösungsmittel, wie z. B. Methanol, Äthanol, Aceton oder Dimethylformamid, in Frage. Aus Haltbarkeitsgründen kann es vorteilhaft sein, die für den Test benötigten Reagentien auf zwei oder mehr Lösungen zu verteilen, die erst bei der eigentlichen Untersuchung vermischt werden.

Zur Herstellung des diagnostischen Mittels in Form eines Lyophilisats im Gesamtgewicht von jeweils etwa 5 - 20 mg, vorzugsweise etwa 10 mg, wird eine Lösung getrocknet, die neben sämtlichen für den Test benötigten Reagentien übliche Gerüstbildner, wie z. B. Polyvinylpyrrolidon, und eventuell weitere Füllstoffe, wie z. B. Mannit, Sorbit oder Xylit enthält.

Ein diagnostisches Mittel in Form von Pulvermischungen oder Reagenztabletten läßt sich herstellen, indem die Bestandteile des Testes mit üblichen galenischen Zusatzstoffen versetzt und granuliert werden. Zusatzstoffe dieser Art sind z. B. Zuckeralkohole, wie Mannit, Sorbit oder Xylit, oder andere lösliche inerte Verbindungen, wie Polyethylenglykole oder Polyvinylpyrrolidon. Die Pulvermischungen oder Reagenztabletten weisen im allgemeinen ein Endgewicht von ungefähr 30 bis 200 mg, vorzugsweise 50 bis 80 mg, auf.

Zur Herstellung des diagnostischen Mittels in Form eines Teststreifens wird ein saugfähiger Träger, vorzugsweise Filterpapier, Cellulose oder Kunstfaservlies, mit Lösungen der erforderlichen, üblicherweise zur Herstellung von Teststreifen verwendeten Reagentien in leicht flüchtigen Lösungsmitteln, wie z. B. Wasser, Methanol, Äthanol oder Aceton imprägniert. Dies kann in einem Imprägnierschritt erfolgen. Oft ist es jedoch zweckmäßig, die Imprägnierung in mehreren Schritten durchzuführen, wobei Lösungen eingesetzt werden, die jeweils einen Teil der Bestandteile des diagnostischen Mittels enthalten. So kann beispielsweise in einem ersten Schritt mit einer wässrigen Lösung, die den Puffer und andere wasserlösliche Zusatsstoffe enthält, und dann in einem zweiten Schritt mit einer Lösung, die das γ-GT-Substrat enthält, imprägniert werden. Die fertigen Testpapiere können als solche verwendet werden oder in an sich bekannter Weise an Griffen angeklebt oder vorzugsweise zwischen Kunststoffen und feinmaschigen Netzwerken gemäß DBP 2118455 eingesiegelt werden.

Die nachfolgenden Beispiele zeigen einige der zahlreichen Verfahrensvarianten, die zur Synthese der erfindungsgemäßen Verbindungen beschritten werden können sowie beispielhaft die Verwendung der neuen γ-Glutamyl-4-azoanilide zur Bestimmung der Aktivität von γ-Glutamyltransferase. Sie sollen jedoch nicht eine Einschränkung des Erfindungsgegenstandes bedeuten.

Beispiel 1

2-Amino-5-[[4-(2-cyan-4-nitrophenyl)azo-3-(2-carboxyethyl)phenyl] amino]-5-oxopentansäure, Diammoniumsalz (S1)

a) 3-(3'-Aminophenyl)propionsäure

30 g 3-Nitrobenzaldehyd und 30 g Malonsäure wurden in 60 ml Pyridin bei maximal 50° C solange erwärmt, bis eine klare Lösung entstanden war. Anschließend wurden 3 ml Piperidin zugegeben und die Lösung eine Stunde lang auf 80° C erwärmt. Dann wurde drei Stunden lang bis zum Rückfluß erhitzt. Die Lösung wurde dann in 400 ml Wasser gegossen, welches 50 ml konzentrierte Salzsäure enthielt. Der ausgefallene Feststoff wurde abfiltriert und mit Wasser gewaschen. Er wurde anschließend in 400 ml Wasser, in dem 8 g Natriumhydroxid gelöst waren, aufgelöst, dann wurde vom unlöslichen Rückstand abfiltriert und 3-Nitrozimtsäure durch Ansäuern mit Salzsäure wieder ausgefällt. Die Verbindung wurde aus Äthanol umkristallisiert und ergab 17 g Produkt.

Schmelzpunkt 204° C.

15 g 3-Nitrozimtsäure wurden zusammen mit 0,4 g eines 5 %igen Palladium-auf-Kohle-Katalysators zu 100 ml Äthanol gegeben. Diese Suspension wurde bei Atmosphärendruck und Raumtemperatur mit Wasserstoff reduziert und nach vollständiger Wasserstoffaufnahme wurden weitere 15 g 3-Nitrozimtsäure zugegeben und die Hydrogenierung wiederholt. Der Katalysator wurde anschließend abfiltriert und die Lösung im Vakuum bis zur Trockne eingeengt. Bei Zugabe von 50 ml Diäthyläther zu dem verbleibenden viskosen Öl erfolgte schnelle Kristallisation. Es wurden so 20 g 3-(3'-Aminophenyl)propionsäure als farbloser Feststoff erhalten.

Schmelzpunkt 73° C.

b) Diazotierung von 2-Cyano-4-nitroanilin

Durch Zugabe von 1,4 g Natriumnitrit zu 15 ml 98 %iger Schwefelsäure wurde unter Beibehaltung einer Temperatur von weniger als 10° C eine Lösung von Nitrosylschwefelsäure zubereitet. Nach beendeter Zugabe des Natriumnitrits wurde die Mischung auf 60° C erwärmt und solange gerührt, bis eine Klare Lösung entstanden war. Diese Lösung wurde dann auf unter 20°C abgekühlt und unter kräftigem Rühren langsam mit 0,02 mol des gepulverten 2-Cyano-4-nitroanilin versetzt. Die Zugabe erfolgte dermaßen, daß die Temperatur nicht über 20° C anstieg. Anschließend wurde noch 45 Minuten bei 20° C gerührt, bevor die resultierende Lösung des Diazoniumsalzes für die Azokupplung mit 3(3'-Aminophenyl)-propionsäure verwendet wurde.

c) Azokupplung von 2-Cyano-4-nitrophenyldiazoniumsalz mit 3-(3'-Aminophenyl)propionsäure

0,02 mol 3-(3'-Aminophenyl)propionsäure wurden in 40 ml 10 %iger Schwefelsäure gelöst. Zu dieser Lösung wurden zunächst 50 g Eis und dann unter Rühren und Kühlung auf eine Temperatur unter 5° C 0,02 mol der unter b) hergestellten Diazoniumsalz-Lösung langsam zugegeben. Nach etwa 2 Stunden wurde die Reaktionsmischung auf Raumtemperatur erwärmt und noch weitere 2 Stunden gerührt. Dann wurden 100 ml Wasser und 50 g Eis zugegeben und der ausgefallene Azofarbstoff abfiltriert. Der Feststoff wurde mit Wasser neutral gewaschen und dann in 125 ml Aceton kräftig gerührt. Der Farbstoff wurde abfiltriert und bei 50° C getrocknet.

$\lambda_{max}$ = 488 nm

ε= 11,3 $cm^2 \mu mol^{-1}$

d) N-Phthaloyl-Derivat von S1

1 g des Aminoazofarbstoffes aus c) und 1,5 g N-Phthaloylglutaminsäureanhydrid wurden in 30 ml Essigsäure gelöst. Die Lösung wurde auf 53°C erwärmt und 21 Stunden lang gerührt. Dann wurde die Reaktionsmischung im Vakuum bis auf ein Volumen von 10 ml eingeengt. Diese konzentrierte Lösung wurde in Wasser gegossen und das ausgefallene Produkt abfiltriert.

e) S1

Die unter d) hergestellte N-phthaloylgeschützte Verbindung wurde in 20 ml Methanol bei 20°C mit 1 ml Hydrazinhydrat versetzt. Die Mischung wurde 2 Stunden gerührt. Dann wurde die Mischung mit 50 ml Aceton versetzt und weitere 2 Stunden gerührt. Anschließend wurde 1 ml einer konzentrierten wäßrigen Ammoniaklösung zugegeben, um die N-Glutamylverbindung als Ammoniumsalz auszufällen. Der ausgefallene Feststoff wurde abfiltriert und erneut in einer minimalen Menge destillierten Wassers gelöst. Durch erneute Zugabe von Aceton wurde das Produkt wieder ausgefällt, abfiltriert und getrocknet. Es wurden so 0,92 g S1 erhalten.
$\lambda_{max}$ = 394 nm

Beispiel 2

2-Amino-5-[[4-(2-cyan-4-nitrophenyl)azo-3-(2-carboxymethoxy)-phenyl]-amino]-5-oxopentansäure, Diammoniumsalz (S2)

a) 3-Aminophenoxy-essigsäure

45 g 3-Acetylaminophenol, 28,5 g Chloressigsäure und 24 g Natriumhydroxid wurden in 200 ml Wasser gelöst und unter Rückfluß 30 Minuten lang erhitzt. Dann wurde so lange Wasser abdestilliert, bis erste Kristalle sich abzuscheiden begannen. In diesem Stadium wurde eine Lösung von 14,3 g Chloressigsäure in 100 ml Wasser zugegeben. Daran anschließend wurde eine Lösung von 12 g Natriumhydroxid in 30 ml Wasser zugefügt. Es wurde erneut eine Stunde lang bis zum Rückfluß erhitzt, bevor die heiße Lösung mit ca. 25 ml Salzsäure angesäuert wurde. Nach Abkühlung der Lösung wurde der ausgefallene Feststoff abfiltriert, zweimal mit je 50 ml Wasser gewaschen und getrocknet.
Die so erhaltene 3-Acetylaminophenoxy-essigsäure wurde in einer Mischung von 100 ml Wasser und konzentrierter Salzsäure 15 Minuten lang bis zum Rückfluß erhitzt. Dann wurden 300 ml Wasser zugegeben und die Lösung mit Ammoniaklösung auf einen pH-Wert von 4 - 5 eingestellt. Der ausgefallene Feststoff wurde abfiltriert und mit leicht essigsaurem Wasser gewaschen. Man erhielt so 36 g 3-Aminophenoxy-essigsäure in Form weißer Kristalle.
Schmelzpunkt 220° C.

b) Azokupplung von 2-Cyano-4-nitrophenyldiazoniumsalz mit 3-Aminophenoxy-essigsäure

Die Azokupplung erfolgte analog Beispiel 1 c) mit jeweils 0,02 mol der Reaktionspartner. Der anfallende Azofarbstoff wurde abfiltriert, mit Aceton gewaschen und bei 50° C getrocknet.
$\lambda_{max}$ = 508 nm
$\xi$ = 19,5 $cm^2 \mu mol^{-1}$

c) N-Phthaloyl - Derivat von S2

Die Acylierung des in Beispiel 2 b) hergestellten Aminoazofarbstoffes mit N-Phthaloylglutaminsäureanhydrid erfolgte analog Beispiel 1 d) mit 1 g Farbstoff. Statt 1,5 g N-Phthaloylglutaminsäureanhydrid wurden 1,3 g Acylierungsmittel verwendet. Die Reaktionszeit betrug 28 Stunden.

d) S2

Die in Beispiel 2 c) hergestellte N-Phthaloylgeschützte Verbindung wurde analog Beispiel 1 e) umgesetzt. Man erhielt so 0,14 g S2.
$\lambda_{max}$ = 414 nm

Beispiel 3

5-[(4-Amino-4-carboxy-1-oxobutyl)amino]-2-(4-nitrophenyl)azobenzoesäure, Diammoniumsalz (S3)

a) 4-Nitrophenyldiazoniumsalz

0,02 mol 4-Nitroanilin wurden in einer Mischung aus 10 ml Essigsäure und 10 ml Salzsäure erwärmt, bis eine klare Lösung entstanden war. Diese Lösung wurde dann unter kräftigem Rühren auf eine Mischung von 40 g Eis und 10 ml Wasser gegossen. Unmittelbar anschließend wurde auf einmal eine Lösung von 1,45 g Natriumnitrit in 10 ml Wasser, welche vorher auf 0° C abgekühlt worden war, zugegeben. Man rührte noch weitere 15 Minuten und zersetzte dann überschüssige salpetrige Säure mit etwas Harnstoff. Die klare Lösung wurde ohne weitere Aufarbeitung zur anschließenden Azokupplung verwendet.

b) Azokupplung von 4-Nitrophenyldiazoniumsalz mit 3-Aminobenzoesäure

Zuerst wurde das N-Methansulfonat von 3-Aminobenzoesäure hergestellt. Hierzu wurden 0,02 mol des Amins in 4 ml Wasser, welches 0,8 g Natriumhydroxid enthielt, gelöst. Diese Lösung wurde dann zu einer Lösung von 1,9 g Natriumbisulfit ($Na_2S_2O_5$) und 1,6 ml einer 40 %igen wässrigen Formaldehyd-Lösung in 4 ml Wasser gegeben. Die Mischung wurde 1 Stunde lang auf 90° C erwärmt und dann auf Raumtemperatur abgekühlt.

Die so hergestellte Lösung des N-Methansulfonats wurde mit 100 ml Eiswasser verdünnt. Hierzu wurden 10 g Natriumhydrogencarbonat gegeben. Die in Beispiel 3 a) hergestellte Diazoniumsalzlösung wurde dann langsam, über einen Zeitraum von 30 Minuten, zugefügt, wobei darauf geachtet wurde, daß die Temperatur der Reaktionsmischung immer unter 10° C blieb. Anschließend wurde noch 1 Stunde bei Raumtemperatur weitergerührt.

Die Lösung des N-Methansulfonats des Aminoazofarbstoffs wurde dann auf 80° C erwärmt und mit einer Lösung von 7 g Natriumhydroxid in 16 ml Wasser versetzt. So wurde ein pH von 11-12 erzielt. Die Lösung wurde 15 Minuten lang bei 80° C gehalten, dann auf Raumtemperatur abgekühlt und mit konzentrierter Salzsäure auf pH 3 angesäuert. Der daraufhin ausfallende Farbstoff wurde abfiltriert, mit Wasser gewaschen und getrocknet.
$\lambda_{max}$ = 404 nm
$\xi$ = 7,0 $cm^2 \mu mol^{-1}$

c) N-Phthaloyl-Derivat von S3

1 g des Aminoazofarbstoffes und 1,8 g N-Phthaloylglutaminsäureanhydrid wurden in 40 ml siedendem Aceton 24 Stunden lang gerührt. Dann wurde die Reaktionsmischung im Vakuum bis zur Trockne eingeengt. Der Rückstand wurde ohne weitere Aufarbeitung zur Schutzgruppenentfernung verwendet.

d) S3

Die Entfernung der Schutzgruppe bei der in Beispiel 3 c) hergestellten Verbindung erfolgte analog den Beispielen 1 e) und 2 d). Es wurden, 0,18 g Produkt erhalten.
$\lambda_{max}$ = 314 nm

Beispiel 4

2-Amino-5-[[4-(2,5-dichlor-4-nitrophenyl)azo-3-(carboxymethoxy)-phenyl]amino]-5-oxopentansäure, Diammoniumsalz (S4)

a) 2,5-Dichloro-4-nitrophenyldiazoniumsalz

2,5-Dichloro-4-nitroanilin wurde analog Beispiel 3 a) diazotiert.

b) Azokupplung von 2,5-Dichloro-4-nitrophenyldiazonumsalz mit 3-Aminophenoxy-essigsäure

Die Azokupplung des in a) hergestellten Diazoniumsalzes mit 3-Aminophenoxy-essigsäure wurde analog der in den Beispielen 1 c) und 2 b) beschriebenen Methode mit jeweils 0,02 mol der Reaktionspartner durchgeführt. Der anfallende Azofarbstoff wurde abfiltriert, mit Aceton gewaschen und bei 50° C getrocknet.
$\lambda_{max}$ = 460 nm
$\xi$ = 13,4 $cm^2 \mu mol^{-1}$

c) N-Phthaloyl - Derivat von S4

Die Acylierung des Aminoazofarbstoffes aus b) erfolgte analog Beispiel 1 d) mit 1 g Farbstoff. Statt 1,5 g N-Phthaloylglutaminsäureanhydrid wurden 1,2 g verwendet. Die Reaktionsmischung wurde 5 Minuten zum Rückfluß erhitzt.

d) S4

Die unter c) hergestellte Verbindung wurde analog den Beispielen 1 e), 2 d) oder 3 d) umgesetzt. Es wurden 0,26 g S4 erhalten.
$\lambda_{max}$ = 402 nm

Beispiel 5

2-Amino-5-[[3-(2-carboxyethyl)-4-(2,4-dicyan-3-methyl-5-thienyl)-azophenyl]amino]-5-oxopentansäure, Diammoniumsalz (S5)

a) Diazotierung von 2-Amino-3,5-dicyano-4-methylthiophen

2-Amino-3,5-dicyano-4-methylthiophen wurde analog Beispiel 1 b) diazotiert.

b) Azokupplung des Diazoniumsalzes von 2-Amino-3,5-dicyano-4-methylthiopen mit 3-(3'-Aminophenyl)propionsäure

Die Azokupplung erfolgte analog Beispiel 1 c) mit jeweils 0,02 mol der Reaktionspartner. Der anfallende Azofarbstoff wurde abfiltriert, mit Aceton gewaschen und bei 50° C getrocknet.
$\lambda_{max}$ = 530 nm
$\xi$ = 14,3 $cm^2\mu mol^{-1}$

c) N-Phthaloyl - Derivat von S5

Die Acylierung des Azofarbstoffes aus b) erfolgte analog Beispiel 1 d) mit 1 g Farbstoff. Statt 1,5 g N-Phthaloylglutaminsäureanhydrid wurde jedoch nur 1 g verwendet. Die Reaktionsmischung wurde 2 Minuten bis zum Rückfluß erhitzt.

d) S5

Die Entfernung der Schutzgruppe bei der in c) hergestellten Verbindung erfolgte analog den Beispielen 1 e) und 2 d). Es wurden 0,14 g S5 erhalten.
$\lambda_{max}$ = 424 nm

Beispiel 6

2-Amino-5-[[3-carboxymethoxy-4-(2,4-dicyan-3-methyl-5-thienyl)azophenyl]amino]-5-oxopentansäure, Diammoniumsalz (S6)

a) Azokupplung des Diazoniumsalzes von 2-Amino-3,5-dicyano-4-methylthiophen mit 3-Aminophenoxy-essigsäure

Die Azokupplung des Diazoniumsalzes von 2-Amino-3,5-dicyano-4-methylthiophen aus Beispiel 5 a) mit 3-Aminophenoxy-essigsäure aus Beispiel 2 a) erfolgte analog Beispiel 5 b) mit jeweils 0,02 mol der Reaktionspartner. Der anfallende Aminoazofarbstoff wurde abfiltriert, mit Aceton gewaschen und bei 50° C getrocknet.
$\lambda_{max}$ = 538 nm
$\xi$ = 26,8 $cm^2\mu mol^{-1}$

b) N-Phthaloyl - Derivat von S6

Die Acylierung des Aminoazofarbstoffes aus a) mit N-Phthaloylglutaminsäureanhydrid erfolgte analog Beispiel 5 c) mit 1 g Farbstoff. Statt 1 g N-Phthaloylglutaminsäureanhydrid wurden 1,2 g verwendet.

c) S6

Die N-Phthaloyl-Schutzgruppe der in b) hergestellten Verbindung wurde analog den Beispielen 1 - 5 entfernt. Es wurden 0,84 g S6 erhalten.
$\lambda_{max}$ = 448 nm

Beispiel 7

2-Amino-5-[[4-(5-carboxy-4-methyl-2-thiazolyl)-3-methyl-azophenyl] amino]-5-oxopentansäure, Diammoniumsalz (S7)

a) Diazotierung von 2-Amino-5-carboxy-4-methylthiazol

Durch Zugabe von 1,4 g Natriumnitrit zu 6 ml 98 %iger Schwefelsäure wurde eine Nitrosylschwefelsäure-Lösung hergestellt. Bei Zugabe des Natriumnitrits wurde darauf geachtet, daß die Temperatur in der Lösung immer unter 10° C blieb. Die Mischung wurde dann anschließend auf 60° C erwärmt und solange gerührt, bis die Lösung klar war. Diese Lösung wurde dann bei etwa 30° C gehalten, um Kristallisation zu verhindern.
Zu einer Mischung von 21 ml 77 %iger Schwefelsäure und 12 ml Wasser wurde eine Lösung von 0,02 mol 2-Amino-5-carboxy-4-methylthiazol in einer Mischung von 2 ml 77 %iger Schwefelsäure und 6 ml Wasser gegeben. Die resultierende Lösung wurde auf - 10° C gekühlt. Hierzu wurde langsam die Nitrosylschwefelsäure-Lösung gegeben, wobei darauf geachtet wurde, daß die Temperatur in der Lösung immer unter - 10° C blieb. Anschließend wurde bei dieser Temperatur 30 Minuten und anschließend bei - 5° C weitere 30 Minuten gerührt. Dann wurde die Lösung mit einer Mischung von 100 g Eis und 100 ml Wasser verdünnt. Überschüssige salpetrige Säure wurde durch Zusatz von Harnstoff zersetzt. Diese so erhaltene Diazoniumsalz-Lösung wurde unmittelbar zur Azokupplung verwendet.

b) Azokupplung des Diazoniumsalzes von 2-Amino-5-carboxy-4-methyl-thiazol mit 3-Methylanilin

Analog der in den Beispielen 1, 2, 4, 5 und 6 beschriebenen Methode wurde die Azokupplung des Diazoniumsalzes von 2-Amino-5-carboxy-4-methylthiazol mit 3-Methylanilin durchgeführt, wobei jeweils 0,02 mol der Reaktionspartner eingesetzt wurden. Der anfallende Azofarbstoff wurde abfiltriert, mit Aceton gewaschen und bei 50° C getrocknet.
$\lambda_{max}$ = 495 nm
$\xi$ = 19,7 $cm^2\mu mol^{-1}$

c) N-Phthaloyl - Derivat von S7

Die Acylierung des in b) erhaltenen Aminoazofarbstoffes mit N-Phthaloylglutaminsäureanhydrid erfolgte analog der in Beispiel 1 d) beschriebenen Methode. Hierbei wurde 1 g Farbstoff mit 1,2 g N-Phthaloylglutaminsäureanhydrid bei Raumtemperatur 48 Stunden lang gerührt.

d) S7

Die Entfernung der N-Phthaloyl-Schutzgruppe bei der in c) hergestellten Verbindung erfolgte analog der in den Beispielen 1 - 6 genannten Methode. Es wurden 0,21 g Produkt erhalten.
$\lambda_{max}$ - 412 nm

Beispiel 8

2-Amino-5-[[3-(2-carboxyethyl)-4-(5-cyan-4-methyl-2-thiazolyl)azophenyl]amino]-5-oxopentansäure, Diammoniumsalz (S8)

a) Azokupplung des Diazoniumsalzes von 2-Amino-5-cyano-4-methyl-thiazol mit 3-(3'-Aminophenyl)propionsäure

Das analog Beispiel 7 a) hergestellte Diazoniumsalz von 2-Amino-5-cyano-4-methylthiazol wurde analog der in Beispiel 7 b) genannten Methode mit 3-(3'-Aminophenyl)propionsäure gekuppelt. Hierbei wurden jeweils 0,02 mol der Reaktionspartner eingesetzt. Der anfallende Aminoazofarbstoff wurde abfiltriert, mit Aceton gewaschen und bei 50° C getrocknet.
$\lambda_{max} = 520$ nm
$\xi$ - 29,7 $cm^2\mu mol^{-1}$

b) N-Phthaloyl - Derivat von S8

1 g des in a) erhaltenen Aminoazofarbstoffes wurde mit 1 g N-Phthaloylglutaminsäureanhydrid in einer Mischung von 40 ml Aceton und 10 ml Essigsäure 18 Stunden lang zum Rückfluß erhitzt. Anschließend wurde das Lösungsmittel im Vakuum entfernt. Der Rückstand wurde direkt, ohne weitere Reinigung der Schutzgruppen-Entfernung unterzogen.

c) S8

Die N-Phthaloyl-Schutzgruppe der in b) hergestellten Verbindung wurde entsprechend der in den Beispielen 1 - 7 genannten Methode entfernt. Es wurden 0,84 g S8 erhalten.
$\lambda_{max} = 420$ nm

Beispiel 9

2-Amino-5-[[3-(2-carboxyethyl)-4-(2-thiazolyl)azophenyl]amino]-5-oxopentansäure, Diammoniumsalz (S9)

a) Azokupplung des Diazoniumsalzes von 2-Aminothiazol mit 3-(3'-Aminophenyl)propionsäure

Die Azokupplung des analog Beispiel 7 a) hergestellten Diazoniumsalzes von 2-Aminothiazol mit 3-(3'-Aminophenyl)-propionsäure erfolgte analog der in den Beispielen 1, 2, 5, 6 ,7 und 8 genannten Methode. Hierbei wurden jeweils 0,02 mol der Reaktionspartner eingesetzt. Der anfallende Aminoazofarbstoff wurde abfiltriert, mit Aceton gewaschen und bei 50° C getrocknet.
$\lambda_{max} = 470$ nm
$\xi = 17{,}0$ $cm^2\mu mol^{-1}$

b) N-Phthaloyl - Derivat von S9

Die Acylierung des in a) erhaltenen Aminoazofarbstoffes mit N-Phthaloylglutaminsäureanhydrid erfolgte analog dem in Beispiel 7 c) angegebenen Verfahren mit 1 g Farbstoff. Statt mit 1,2 g des Acylierungsmittels wurde der Farbstoff 24 Stunden lang bei Raumtemperatur mit 1,1 g N-Phthaloylglutaminsäureanhydrid gerührt.

c) S9

Die Entfernung der N-Phthaloyl-Schutzgruppe bei der in b) hergestellten Verbindung erfolgte analog der in den Beispielen 1 - 8 genannten Methode. Es wurden so 0,25 g S9 erhalten.
$\lambda_{max} = 394$ nm

Beispiel 10

2-Amino-5-[[3-(2-carboxylatoethyl)-4-(3-methylthiazolium-2-yl)azophenyl]amino]-5-oxopentansäure, Ammoniumsalz (S10)

1 g der in Beispiel 9 c) erhaltenen Verbindung wurden in einer Mischung von 20 ml Chlorbenzol und 20 ml Dichlormethan gelöst. Zu dieser Lösung wurden 2 ml säurefreies Dimethylsulfat gegeben. Um sicherzugehen, daß das verwendete Dimethylsulfat frei von Säurespuren war, wurde nur solches Dimethylsulfat verwendet, welches unmittelbar zuvor mit Wasser gewaschen und anschließend über Natriumsulfat getrocknet worden war. Die Reaktionsmischung wurde 24 Stunden lang bei Raumtemperatur gerührt. Dann wurde von ausgefallenem Feststoff abfiltriert und der Feststoff dann zunächst mit wenig Dichlormethan und dann mit wenig Aceton gewaschen. Anschließend wurde der Feststoff mit einer kleinen Menge Methanol, welches zwei Tropfen Essigsäure enthielt, versetzt und zwei Stunden lang gerührt. Dann

wurde der Feststoff abfiltriert, mit einer kleinen Menge Methanol gewaschen und abschließend getrocknet. Es wurden so 0,23 g S10 erhalten.
$\lambda_{max}$ (Substrat) = 402 nm
$\lambda_{max}$ (Farbstoff) = 486 nm
$\phi = 15,5\ cm^2 \mu mol^{-1}$

Beispiel 11

2-Amino-5-[[3-carboxymethoxy-4-(6-nitro-2,1-benzisothiazol-3-yl)azophenyl]amino]-5-oxopentansäure, Diammoniumsalz (S11)

a) Diazotierung von 3-Amino-5-nitrobenzisothiazol

Durch Zugabe von 0,75 g Natriumnitrit zu 10 ml 98 %iger Schwefelsäure bei einer Temperatur unter 10° C wurde eine Lösung von Nitrosylschwefelsäure hergestellt. Die Mischung wurde auf 60° C erhitzt und solange gerührt, bis eine klare Lösung entstanden war.

0,01 mol 3-Amino-5-nitrobenzisothiazol wurde in 10 ml 85 %iger Schwefelsäure gelöst und die Lösung auf unter 10° C abgekühlt. Zu dieser Aminlösung wurde unter kräftigem Rühren und Aufrechterhaltung einer Temperatur unter 10° C die gekühlte Nitrosylschwefelsäurelösung zugegeben. Nach 2 1/2 Stunden wurde überschüssige salpetrige Säure durch Zugabe von Harnstoff zersetzt. Die so erhaltene Diazoniumsalzlösung wurde ohne weitere Aufarbeitung für die Azokupplung verwendet.

b) Azokupplung des Diazoniumsalzes von 3-Amino-5-nitrobenzisothiazol mit 3-Aminophenoxyessigsäure

0,01 mol 3-Aminophenoxy wurden in 10 ml einer Mischung von 1 Volumenteil Propionsäure und 5 Volumenteilen Essigsäure gelöst. Zu dieser Lösung wurden 20 g Eis und 80 ml Wasser gegeben. Anschließend wurden 20 g Natriumacetate-Trihydrat zugefügt. Zu dieser Mischung gab man die in a) hergestellte Diazoniumsalz-Lösung langsam und unter Kühlung zu, wobei darauf geachtet wurde, daß die Temperatur immer unter 10° C blieb. Um einen pH-Wert größer als 4 aufrecht zu erhalten, wurde - falls notwendig - weiteres Natriumacetat zugefügt. Nach 12stündigem Rühren bei Raumtemperatur wurde die Mischung mit 500 ml heißem Wasser verdünnt und der daraufhin ausgefallene Azofarbstoff abfiltriert, mit Wasser gewaschen und getrocknet. Die Reinigung erfolgte durch gründliches Verreiben mit einer kleinen Menge Aceton und anschließende Filtration und Trocknung des so erhaltenen Farbstoffes.
$\lambda_{max}$ = 560 nm
$\xi = 2,2\ cm^2 \mu mol^{-1}$

c) N-Phthaloyl - Derivat von S11

1 g des in b) hergestellten Azofarbstoffes wurde analog Beispiel 8 b) mit N-Phthaloylglutaminsäureanhydrid acyliert. Statt 18 Stunden wurde die Reaktionsmischung in diesem Fall aber nur 7 Stunden lang zum Rückfluß erhitzt.

d) S11

Die in c) hergestellte N-Phthaloylgeschützte Verbindung wurde analog der in den vorangegangenen Beispielen beschriebenen Methode mit Hydrazin umgesetzt, um die Phthaloyl-Schutzgruppe zu entfernen. Es wurden 0,70 g Produkt erhalten.
$\lambda_{max}$ = 453 nm

Beispiel 12

2-Amino-5-[[4-(2-butylthio-1,3,4-thiadiazol-5-yl)-3-carboxymethoxy-azophenyl]amino]-5-oxopentansäure, Diammoniumsalz (S12)

a) Azokupplung des Diazoniumsalzes von 2-Amino-5-(n-butylthio)-1,3,4-thiadiazol mit 3-Aminophenoxyessigsäure

Das analog Beispiel 11 a) hergestellte Diazoniumsalz von 2-Amino-5-(n-butylthio)-1,3,4-thiadiazol wurde mit 3-Amino-phenoxy-essigsäure analog Beispiel 1 c) oder 2 b) gekuppelt. Es wurden jeweils 0,02 mol

der Reaktionspartner eingesetzt. Der anfallende Farbstoff wurde abfiltriert, mit Aceton gewaschen und bei 50° C getrocknet.
$\lambda_{max} = 490$ nm
$\xi = 15{,}2$ $cm^2\mu mol^{-1}$

b) N-Phthaloyl - Derivat von S12

Die Acylierung von 1 g des in a) erhaltenen Aminoazofarbstoffs mit 1,2 g N-Phthaloylglutaminsäureanhydrid erfolgte analog den Beispielen 7 c) oder 9 b). Die Reaktionsmischung wurde 1 Minute zum Rückfluß erhitzt.

c) S12

Die Entfernung der Schutzgruppe bei der in b) erhaltenen Verbindung wurde analog der in den vorangehenden Beispielen genannten Methode durchgeführt. Es wurden so 0,32 g Produkt erhalten.
$\lambda_{max} = 424$ nm

Beispiel 13

2-Amino-5-[[3-carboxymethoxy-4-(6-ethoxy-2-benzothiazolyl)azophenyl]-amino]-5-oxopentansäure, Diammoniumsalz (S13)

a) Azokupplung des Diazoniumsalzes von 2-Amino-6-ethoxy-benzthiazol mit 3-Aminophenoxy-essigsäure

Das analog Beispiel 7 a) hergestellte Diazoniumsalz von 2-Amino-6-ethoxybenzthiazol wurde analog der in Beispiel 7 b) genannten Methode mit 3-Aminophenoxy-essigsäure gekuppelt. Es wurden hierzu jeweils 0,02 mol der Reaktionspartner eingesetzt. Der anfallende Farbstoff wurde abfiltriert, mit Aceton gewaschen und bei 50° C getrocknet.
$\lambda_{max}$ - 494 nm
$\xi = 24{,}0$ $cm^2\mu mol^{-1}$

b) N-Phthaloyl - Derivat von S13

Die Acylierung von 1 g des unter a) hergestellten Aminoazofarbstoffs mit 0,8 g N-Phthaloylglutaminsäureanhydrid erfolgte analog der in Beispiel 9 b) genannten Methode. Das Reaktionsgemisch wurde 2 Minuten bis zum Rückfluß erhitzt und anschließend im Vakuum bis zur Trockne eingeengt. Der Rückstand wurde ohne weitere Aufarbeitung für die Schutzgruppenentfernung verwendet.

c) S13

Die Entfernung der N-Phthaloyl-Schutzgruppe bei der unter b) hergestellten Verbindung erfolgte analog der in Beispiel 1 e) beschriebenen. Es wurden so 0,9 g S13 erhalten.
$\lambda_{max} = 449$ nm

Beispiel 14

Bestimmung der Aktivität von $\gamma$-Glutamyltransferase

a) Reagenz

21, 78 g Glycylglycin werden in ca. 900 ml destilliertem Wasser gelöst, mit 2 N Natronlauge auf pH 7,9 eingestellt, die für eine Konzentration von 1 mmol/1 berechnete Menge Substrat darin gelöst und die Lösung mit destilliertem Wasser auf 1000 ml aufgefüllt.
Endkonzentrationen im Test:
Glycylglycin 150 mmol/1, pH 7,9
Substrat 1 mmol/1

b) Durchführung der Messung

2,0 ml des unter a) beschriebenen Reagenz werden in eine Küvette pipettiert, auf 25° C temperiert und mit 0,2 ml Probe gemischt. Die Extinktionszunahme pro Minute wird bei einer geeigneten Messwellenlänge bestimmt.

c) Berechnung der Aktivität

Eine internationale Einheit (U) ist die Enzymaktivität, die bei 25° C 1 µmol Substrat pro 1 Minute umsetzt. Die Aktivität der γ-Glutamyltransferase in U/1 in der Probe wird nach der allgemeinen Formel berechnet:

$$U/1 = \frac{\Delta E/min. * V * 1000}{\varepsilon * v * d}$$

wobei
ΔE/min = Extinktionszunahme bei geeigneter Meßwellenlänge/Minute
V = Ansatzvolumen in ml
v = Probevolumen in ml
ξ = Extinktionskoeffizient in $cm^2 \mu mol^{-1}$
d = Schichtdicke der Küvette in cm

d) Ergebnisse

Bei Untersuchung des gleichen Kontrollserums mit unterschiedlichen Substraten wurden die in nachstehender Tabelle stehenden Aktivitäten für γ-GT ermittelt.

| Substanz | Meßwellen länge (nm) | Extinktions koeffizient ($cm^2/umol^{-1}$) | U/1 bei 25° C |
|---|---|---|---|
| S 1 | 546 | 7.8 | 144 |
| S 5 | 578 | 8.9 | 77 |
| S 8 | 546 | 25.2 | 101 |
| S 9 | 546 | 2.8 | 160 |

e) Vergleich

Zum Vergleich mit den unter d) gefundenen Werten wurde das gleiche Kontrollserum mit dem bisher üblichen Substrat L-γ-Glutamyl-3-carboxy-4-nitroanilid (L-Glupa C) untersucht.

| Substanz | Meßwellen länge (nm) | Extinktions koeffizient ($cm^2/uMol$) | U/1 bei 25° C |
|---|---|---|---|
| (L-Glupa-C) | 405 | 9.5 | 128 |

Beispiel 15

Bestimmung der Aktivität von γ-Glutamyltransferase bei 25° C und 37° C mit S1 und L-γ-Glutamyl-3-carboxy-4-nitroanilid (L-Glupa C)

Analog Beispiel 14wurden in drei Gemischen von Humanseren in drei verschiedenen Aktivitätsbereichen sowie in einem Kontrollserum die Aktivitäten von γ-Glutamyltransferase bei 25° C und 37° C bestimmt. Substrate waren L-Glupa-C und S1. Es wurden folgende Werte gefunden:

| Probe | mit Glupa-C | | mit S1 | |
|---|---|---|---|---|
| | 25° C | 37° C | 25° C | 37° C |
| | U/l | U/l | U/l | U/l |
| Humanserumpool 1 | 35 | 57 | 42 | 96 |
| " 2 | 63 | 106 | 76 | 176 |
| " 3 | 190 | 323 | 220 | 490 |
| Kontrollserum PPU 516 | 139 | 211 | 134 | 268 |

**Patentansprüche**

1. γ-Glutamyl-4-azoanilide der allgemeinen Formel I:

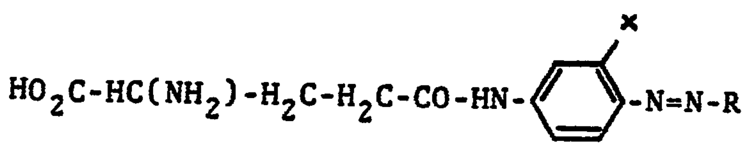

(I)

in der
X eine Gruppe $-(CH_2)_m-CO_2H$, $-O(CH_2)_n-CO_2H$, oder einen Alkylrest,
R eine p-Nitrophenylgruppe, die gegebenenfalls ein- oder mehrfach durch Halogen, einen Cyanorest, einen Alkoxyrest, eine Hydroxygruppe, einen Alkylrest, einen Carboxyalkylrest, eine Aminogruppe, einen Alkylamino- oder Dialkylaminorest substituiert sein kann, wobei die Substituenten gleich oder verschieden sein können,
ein Thiophenrest, der gegebenenfalls ein- oder mehrfach durch einen Cyano-, Nitro-, Alkyl- oder Carboxyalkylrest substituiert sein kann, wobei die Substituenten gleich oder verschieden sein können,
ein Thiazolrest, der gegebenenfalls ein- oder mehrfach durch einen Cyano-, Carboxyl- oder Alkylrest substituiert sein kann, wobei die Substituenten gleich oder verschieden sein können,
einen Benzothiazolrest, der gegebenenfalls ein- oder mehrfach durch einen Alkoxyrest substituiert sein kann,
einen Benzoisothiazolrest, der gegebenenfalls ein- oder mehrfach durch eine Nitrogruppe substituiert sein kann oder
einen N-Alkylthiazolrest oder
einen 1,3,4-Thiodiazolrest, der gegebenenfalls ein- oder mehrfach durch eine Alkylthiogruppe substituiert sein kann
bedeuten, wobei
m eine ganze Zahl von 0 bis 4 und
n eine ganze Zahl von 1 bis 4 darstellen.
sowie deren Alkali-, Erdalkali- und Ammoniumsalze.

2. Verfahren zur Herstellung der Verbindungen der allgemeinen Formel I:

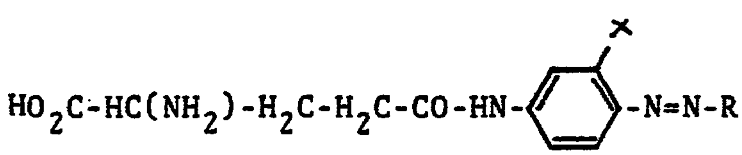

(I)

in der
X eine Gruppe $-(CH_2)_m-CO_2H$, $-O(CH_2)_n-CO_2H$ oder einen Alkylrest,
R eine p-Nitrophenylgruppe, die gegebenenfalls ein- oder mehrfach durch Halogen, einen Cyanorest, einen Alkoxyrest, eine Hydroxygruppe, einen Alkylrest, einen Carboxyalkylrest, eine Aminogruppe, einen Alkylamino- oder Dialkylaminorest substituiert sein kann, wobei die Substituenten gleich oder verschieden sein können,
ein Thiophenrest, der gegebenenfalls ein- oder mehrfach durch einen Cyano-, Nitro-, Alkyl- oder Carboxyalkylrest substituiert sein kann, wobei die Substituenten gleich oder verschieden sein können,

ein Thiazolrest, der gegebenenfalls ein- oder mehrfach durch einen Cyano-, Carboxyl- oder Alkylrest substituiert sein kann, wobei die Substituenten gleich oder verschieden sein können,
einen Benzothiazolrest, der gegebenenfalls ein- oder mehrfach durch einen Alkoxyrest substituiert sein kann,
einen Benzoisothiazolrest, der gegebenenfalls ein- oder mehrfach durch eine Nitrogruppe substituiert sein kann oder
einen N-Alkylthiazolrest oder
einen 1,3,4-Thiodiazolrest, der gegebenenfalls ein- oder mehrfach durch eine Alkylthiogruppe substituiert sein kann
bedeuten, wobei
m eine ganze Zahl von 0 bis 4 und
n eine ganze Zahl von 1 bis 4 darstellen
sowie deren Alkali-, Erdalkali- und Ammoniumsalze,
dadurch gekennzeichnet, daß Amine der allgemeinen Formel II

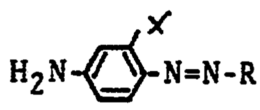

(II)

in der X und R die in der allgemeinen Formel I angegebene Bedeutung haben, mit N-Phthaloylglutaminsäureanhydrid zu N-Phthaloylgeschützten γ-Glutamyl-Derivaten der allgemeinen Formel III

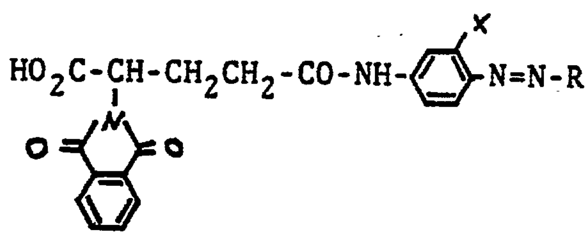

(III)

umgesetzt werden und abschließend die N-Phthaloyl-Schutzgruppe entfernt wird, wobei die hierbei entstehenden Carbonsäuren nach bekannten Methoden in gewünschte Salze überführt werden können oder je nach gewählten Verfahrensbedingungen Salze auch direkt erhalten werden können.

3. Verfahren gemäß Anspruch 2, dadurch gekennzeichnet, daß die N-Phthaloyl-Schutzgruppe mit Hydrazin entfernt wird.

4. Verwendung der Verbindungen gemäß Anspruch 1 als Substrat für die Bestimmung der Aktivität des Enzyms γ-Glutamyltransferase.

5. Diagnostisches Mittel zur Bestimmung der Aktivität des Enzyms γ-Glutamyltransferase enthaltend ein oder mehrere chromogene Substrate, eine Aminosäure oder ein Peptid, ein geeignetes Puffersystem sowie gegebenenfalls weitere üblicherweise verwendete Hilfsstoffe, dadurch gekennzeichnet, daß als chromogene Substrate γ-Glutamyl-4-azoanilide gemäß Anspruch 1 eingesetzt werden.

## Claims

1. γ-Glutamyl-4-azoanilides of the general formula I

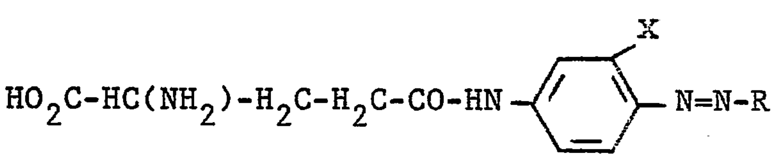

(I)

in which X signifies a group $-(CH_2)_mCO_2H$ or $-O-(CH_2)_nCO_2H$ or an alkyl radical, R a p-nitrophenyl group which can possibly be substituted one or more times by halogen, a cyano radical, an alkoxy radical, a hydroxyl group, an alkyl radical, a carboxyalkyl radical, an amino group, an alkylamino or dialkylamino radical, whereby the substituents can be the same or different, a thiophene radical which can possibly be substituted one or more times by a cyano, nitro, alkyl or carboxyalkyl radical, whereby the substituents can be the same or different; a thiazole radical which can possibly be substituted one or more times by a cyano, carboxyl or alkyl radical, whereby the substituents can be the same or different; a benzothiazole radical which can possibly be substituted one or more times by an alkoxy radical, a benzoisothiazole radical which can possibly be substituted one or more times by a nitro group, or an N-alkylthiazole radical or is a 1,3,4-thiodiazole radical which can possibly be substituted one or more times by an alkylthio group, whereby m represents a whole number of 0 to 4 and n a whole number of 1 to 4; as well as their alkali metal, alkaline earth metal and ammonium salts.

2. Process for the preparation of compounds of the general formula I

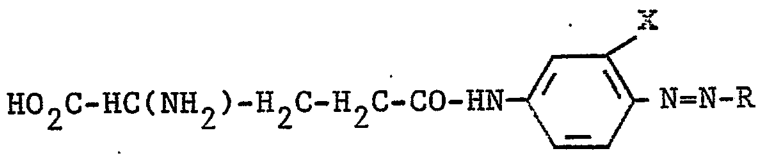 (I)

in which X signifies a group $-(CH_2)_m-CO_2H$ or $-O-(CH_2)_n-CO_2H$ or an alkyl radical, R a p-nitrophenyl group which can possibly be substituted one or more times by halogen, a cyano radical, an alkoxy radical, a hydroxyl group, an alkyl radical, a carboxyalkyl radical, an amino group, an alkylamino or dialkylamino radical, whereby the substituents can be the same or different; a thiophene radical which can possibly be substituted one or more times by a cyano, nitro, alkyl or carboxyalkyl radical, whereby the substituents can be the same or different; a thiazole radical, which can possibly be substituted one or more times by a cyano, carboxyl or alkyl radical, whereby the substituents can be the same or different, a benzothiazole radical which can possibly be substituted one or more times by an alkoxy radical, a benzoisothiazole radical which can possibly be substituted one or more times by a nitro group, or R an N-alkylthiazole radical or a 1,3,4-thiodiazole radical which can possibly be substituted one or more times by an alkylthio group, whereby m represents a whole number of 0 to 4 and n a whole number of 1 to 4; as well as of their alkali metal, alkaline earth metal and ammonium salts thereof, characterised in that amines of the general formula II

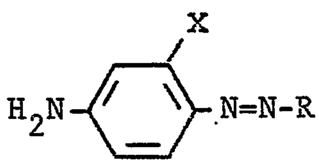 (II)

in which X and R have the meanings given in general formula I, are reacted with N-phthaloylglutamic acid anhydride to give N-phthaloyl-protected-γ-glutamyl derivatives of the general formula III

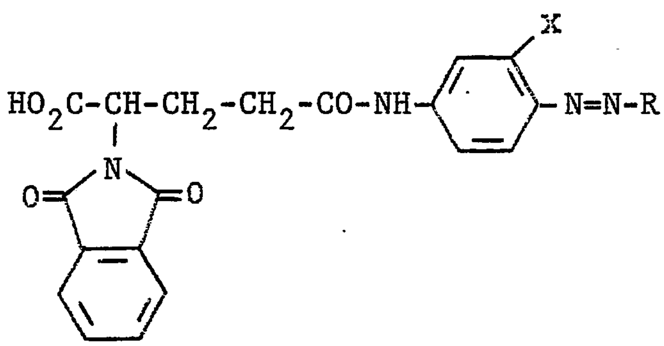 (III)

and subsequently the N-phthaloyl protective group is removed, whereby the hereby resulting carboxylic acids can be converted according to known methods into desired salts or, depending upon the chosen process conditions, salts can also be obtained directly.

3. Process according to claim 2, characterised in that the N-phthaloyl protective group is removed with hydrazine.

4. Use of the compounds according to claim 1 as substrate for the determination of the activity of the enzyme γ-glutamyl transferase.

5. Diagnostic agent for the determination of the activity of the enzyme γ-glutamyl transferase, containing one or more chromogenic substrates, an amino acid or a peptide, a suitable buffer system, as well as possibly further conventionally used adjuvants, characterised in that, as chromogenic substrates there are used γ-glutamyl-4-azoanilides according to claim 1.

**Revendications**

1. γ-Glutamyl-4-azoanilide de formule générale I:

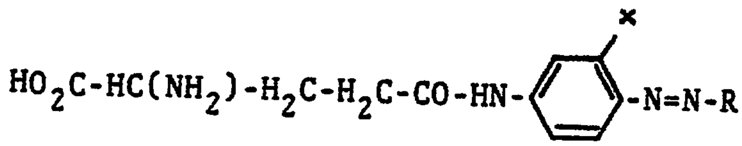

(I)

dans laquelle
X représente un groupe $-(CH_2)_m-CO_2H$, $-O(CH_2)_n-CO_2H$ ou un groupe alkyle,
R représente un groupe p-nitrophenyle, qui peut être éventuellement substitué une ou plusieurs fois par un atome d'halogène, un groupe cyano, un groupe alcoxy, un groupe hydroxy, un groupe alkyle, un groupe carboxyalkyle, un groupe amino, un groupe alkylamino ou un groupe dialkylamino, les substituants pouvant être identiques ou différents,
un groupe thiophène, qui peut être éventuellement substitué une ou plusieurs fois par un groupe cyano, un groupe nitro, un groupe alkyle ou un groupe carboxyalkyle, les substituants pouvant être identiques ou différents,
un groupe thiazole, qui peut être éventuellement substitué une ou plusieurs fois par un groupe cyano, un groupe carboxyle ou un groupe alkyle, les substituants pouvant être identiques ou différents,
un groupe benzothiazole, qui peut être éventuellement substitué une ou plusieurs fois par un groupe alcoxy,
un groupe benzoisothiazole, qui peut être éventuellement substitué une ou plusieurs fois par un groupe nitro,
un groupe N-alkylthiazole ou
un groupe 1,3,4-thiodiazole, qui peut être éventuellement substitué une ou plusieurs fois par un groupe alkylthio,
m valant un nombre entier de 0 à 4 et
n valant un nombre entier de 1 à 4,
ainsi que leurs sels de métaux alcalins, alcalino-terreux et d'ammonium.

2. Procédé pour la préparation des composés de formule générale:

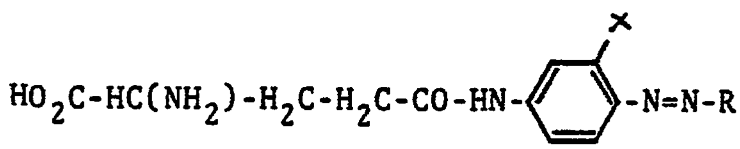

(I)

dans laquelle
X représente un groupe $-(CH_2)_m-CO_2H$, $-O(CH_2)_n-CO_2H$ ou un groupe alkyle,
R représente un groupe p-nitrophényle, qui peut être éventuellement substitué une ou plusieurs fois par un atome d'halogène, un groupe cyano, un groupe alcoxy, un groupe hydroxy, un groupe alkyle, un groupe carboxyalkyle, un groupe amino, un groupe alkylamino ou un groupe dialkylamino, les substituants pouvant être identiques ou différents,
un groupe thiophène, qui peut être éventuellement substitué une ou plusieurs fois par un groupe cyano, un groupe nitro, un groupe alkyle ou un groupe carboxyalkyle, les substituants pouvant être identiques ou différents,
un groupe thiazole, qui peut être éventuellement substitué une ou plusieurs fois par un groupe cyano, un groupe carboxyle ou un groupe alkyle, les substituants pouvant être identiques ou différents,
un groupe benzothiazole, qui peut être éventuellement substitué une ou plusieurs fois par un groupe alcoxy,
un groupe benzoisothiazole, qui peut être éventuellement substitué une ou plusieurs fois par un groupe nitro,
un groupe N-alkylthiazole ou
un groupe 1,3,4-thiodiazole, qui peut être éventuellement substitué une ou plusieurs fois par un groupe alkylthio,
m valant un nombre entier de 0 à 4 et
n valant un nombre entier de 1 à 4,
ainsi que leurs sels de métaux alcalins, alcalino-terreux et d'ammonium, caractérisé en ce que l'on fait réagir des amines de formule générale II

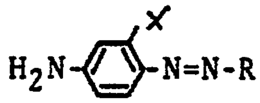

(II)

dans laquelle X et R ont la signification indiquée dans la formule générale I, avec l'anhydride de l'acide N-phtaloylglutamique, pour obtenir des dérivés γ-glutamyliques protégés par un groupe N-phtaloyle, de formule générale III

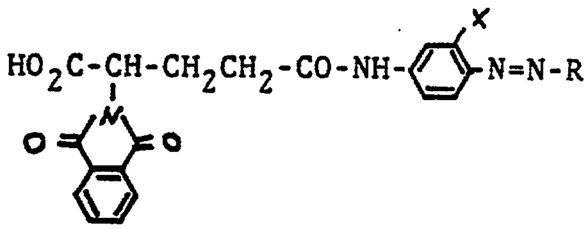

(III)

et ensuite, on élimine le groupe protecteur N-phtaloyle, les acides carboxyliques ainsi produits pouvant être transformés par des procédés connus en leurs sels voulus ou bien, selon les conditions de procédé choisies, les sels peuvent être également obtenus directement.

3. Procédé selon la revendication 2, caractérisé en ce que le groupe de protection N-phtaloyle est éliminé à l'aide d'hydrazine.

4. Utilisation des composés selon la revendication 1, comme substrats pour la détermination de l'activité de l'enzyme-γ-glutamyltransferase.

5. Agent de diagnostic pour la détermination de l'activité de l'enzyme γ-glutamyltransferase, contenant un ou plusieurs substrats chromogènes, un acide amine ou un peptide, un système tampon approprié ainsi qu'éventuellement d'autres adjuvants usuels, caractérisé en ce que comme substrat chromogène, on utilise le γ-glutamyl-4-azoanilide selon la revendication 1.